# EUROPEAN PATENT APPLICATION

(11) **EP 3 692 980 A1**
(43) Date of publication of application: **12.08.2020**
(21) Application number: 20152571.4
(22) Date of filing: 19.01.2020
(51) Int. Cl.: A61K 8/99, A61P 17/00, A61K 35/74

(54) **COMBINED MULTISTAGE MICROBIAL PREPARATIONS AND METHOD OF THEIR APPLICATION**

(30) Priority: 11.02.2019 CZ 20190075
(71) Applicant: BiomCare s.r.o., 26301 Dobris (CZ)
(72) Inventor: BEZOUSKA, Karel, 160 00 Praha 6 (CZ); ENGL, Jan, 40801 Rumburk 1 (CZ); JANDA, Lubomír, 62500 Brno - Bohunice (CZ); NOREK, Adam, 63900 Brno -Stírice (CZ)
(74) Representative: Hak, Roman

(57) **Abstract**

The invention relates to combined multistage microbial preparations that are effective for enhancement of the biological skin barrier and maintenance of healthy skin microbiota. The preparations might be used as cosmetic or medicinal products. Combined multistage microbial cosmetic product are intended for an invigoration of the natural biological reinforcement barrier on the skin and mucosal surfaces weakened in case of chronic problems associated with dysbiosis such as atopic dermatitis, acne, rosacea and vitiligo or acute problems caused by damage of the skin such as burns, cuts and contusions. The preparations comprises a set of compositions for sequential applications of compounds able (1) to dissolve biofilms formed by the pathogenic microorganisms in the skin and suppress the viability of the microorganisms released from the biofilms in the first stage, (2) to calm an inflammation caused by the imbalanced immune system and restore the efficient biological barrier in the second stage, (3) to achieve the restoration of normal physiological microflora disturbed by the dysbiosis in the third stage, and (4) to contribute to the long term nonspecific skin defense and normal consistency and nutrition in the skin. The beneficial substances may be applied onto the skin in the form of oil emulsions, creams, ointments, gels, and other cosmetic or medical formulations.

## Description

### FIELD OF THE INVENTION

The invention relates to combined multistage microbial preparations that are effective for enhancement of the biological skin barrier and maintenance of healthy skin microbiota. The preparations might be used as cosmetic or medicinal products.

### BACKGROUND OF THE INVENTION

The concept of the balances of the microbiota in various parts of human body and evidence for beneficial effects of commensal microorganisms on the health of the skin including the formation of the normal immune response in this location originated in the 1960s and 1970s. The investigations went on at the microbiology/immunology bordlerland. In this early period the main principles of the microbial balances were discovered while their exact mechanistic nature remained unknown due to methodical limitations (detection of individual microorganisms based only on cultivation and biochemical methodologies). The first two decades of the new millennium brought dramatic progress with the new advances in the methods of cultivation independent microorganisms identification based on the PCR amplification of selected gene sequences (most often ITS sequences in the genes for ribosomal RNA) in combination with significant acceleration in DNA sequencing based on the NGS (Next Generation Sequencing) machines and technologies. The second most important factor contributing to our knowledge on the composition of microbial communities in various parts of our body was the Human Microbiome Project guaranteeing sufficient working and financial capacity for thorough investigation of the key issues. Thus, at the beginning of the current decade, the detailed picture of the spectrum of microbiota defining the normal human microbiome and the microbiome associated with various diseases caused by dysbioses could be established (7, 9, 21). Regarding various illnesses on the skin and mucous surfaces, it was shown that weakening of the dominant commensal skin microorganism, the bacterium *Staphylococcus epidermidis* (*S. epidermidis*) can lead to fragility of the biological skin barrier making the skin vulnerable to infections by the feared skin pathogen, e.g. the bacterium *Staphylococcus aureus* (*S. aureus*).

Considering the efforts and costs concentrated into the Human Microbiome Project, the expectations of the general public towards the practical outcomes has been considerable, in particular regarding the new means aimed at management of dysbiosis and illnesses associated with them. However, few of these practical outcomes have appeared so far. Smoragiewicz et al. (22) describe the inhibitory effect of the selected strains of lactobacilli on methicillin-rezistant *S. aureus.* Gallo and Nakatsuji (4) describe a new antimicrobial agent originating from *S. epidermidis* designated as firmocidin. Kleinberg and Zhang (12) developed a simple chemical composition acting as a prebiotic that is able to alter the composition of the skin microbiota by decreasing the proliferation of the dominant skin pathogen *S. aureus* at the expense of the commensal bacterium *S. epidermidis.* Park et al. (20) describe an invention based on strains of *S. epidermidis* disrupting the biofilms formed on the skin by pathogenic microorganisms. Takayama and Yuko (24) describe the modified strains of *S. epidermidis* producing increased amounts of antimicrobial peptides and their use for skin protection. Similar invention by Nakatsuji and Galo (19) suggests antimicrobial therapy based on selected strains of *S. epidermidis* producing hogicidin peptides designated SH-lantibiotics. Similar patent has been filed by Kazue et al. (11).

New critical findings on the skin microbiota have been also the subject of several publications that appeared during the recent years. Naik et al. (17) showed that skin microbiota have an autonomous role in the formation of the local immune system able to eliminate pathogens. The fact that the combination of *S. aureus* infection and prolonged dysbiosis is the driving force of inflammation accompanying atopic dermatitis was proven by Kobayashi et al. (13) using the ADAM17-deficient mice. The relation of the dysbioses of the skin microbiota and various problems of the sensitive skin has been proven repeatedly in many studies, e.g. Kong et al. (14), Ganju et al. (5), and others.

Finally, there are now several studies available that were able to identify particular compound that might be linked to the skin problems. By a vast majority, these compounds are produced by the dominant commensal microorganisms in the skin, such as *S. epidermidis,* and are thus missing in the skin lesions affected by dysbioses. Specific proteinase of the above microorganism cleaves the target proteins after glutamic acid residues (similarly to V8 proteinase) is designated Esp, and its role in the elimination of the pathogenic skin biofilms and the degradation of specific surface receptors of *S. aureus* has been proven (10, 23). Lipoteichoic acid secreted by bacterium *S. epidermidis* has been identified as the key factor calming the skin inflammations (15). It should be noted that the new study showed that natural commensal strains of *S. epidermidis* isolated from skin swabs are not very good producers of this compound, unlike the nontoxic and biofilms non-forming collection strain *S. epidermidis* ATCC_12228 (6). Finally, a mention should be made of the critical role that the antimicrobial peptides produced by some strains of *S. epidermidis,* such as γ-modulin and peptides of the SH-lantibiotic group, have in the maintenance of efficient protection against skin pathogens (1, 2, 18) collaborating with the skin antimicrobial peptides.

All the above described solutions have severe limitations and drawbacks. Most of them have only been tested at the laboratory level and their actual efficacy in humans is thus not supported by adequate tests. The suggested compositions mostly contain viable microorganisms requiring their registration as medicines. However, due to the regulatory hurdles, not even a single medicine with viable microorganisms has entered into the markets making their availability to the affected individuals very limited. The use of autologous strains is often advocated, but limitations in the efficacy mentioned above (6) might represent a serious problem.

### DESCRIPTION OF THE INVENTION

The above mentioned shortcomings are partially or fully overcome by preparations according to the present invention, wherein the findings and principles obtained by the inventors during research, development and practical testing of the product have been explored.

The most important new principle included in the present invention is the multistage solution of the problems of the susceptible persons, whereby it was thought necessary to perform first the thorough cleaning of the skin and removal of pathogenic microorganisms hidden in the biofilms before going on with the calming of the overtly activated immune system. Only after cleaning of the skin, elimination of pathogens, and normalization of microbial imbalance, the prerequisites for the application of substances assuring the long-term stability of skin barriers including its biological component are fulfilled. Active substances in the form of microbial extracts, microbial lysates, or partially purified fractions isolated therefrom are obtained primarily from the commensal skin microorganisms as well as soil and environmental organisms, including those occurring only sporadically at the skin of the modern man. It is possible to imagine the multistage treatment as a series of subsequent treatment waves wherein the following application assures the gradual decrease of the compounds applied in the previous wave while at the same time increasing the concentrations of the substances characteristic for the subsequent wave. The number and timing of the individual waves has been subjected to extensive testing and verification. The system of four subsequent waves (stages) disclosed in this specification represents the maximal modality system with the application of the optimized concentrations of the suitable active compounds, prebiotics and nutrients.

The combined multistage microbial preparation according to the present invention thus contains four different stages, i.e. four different compositions for their sequential application, wherein the first stage composition of the preparation comprises substances able to dissolve biofilms formed by the pathogenic microorganisms in the skin and to suppress the viability of the released pathogenic microorganisms, the second stage composition of the preparation comprises substances able to calm the inflammation caused by imbalanced immune system and to restore the efficient biological barrier, the third stage composition of the preparation comprises substances able to restore the normal skin microflora, and the fourth stage composition of the preparation comprises substances contributing to the long-term protection and stabilization of the physical- chemical barrier of the skin and its nutrition.

The prior-art solutions mentioned in the Background of the Invention are mostly based on the application of the viable microorganisms, which must be followed during their colonization at the application sites. Such preparations and protocols are extremely demanding from the point of view of regulatory and registration procedures. The microorganisms colonizing the skin obviously affect the physiological and immunological processes at the application sites, and thus by definition must fall among medicinal products. The regulatory hurdles connected to the life biotherapeutical products discourage the applicants from massive investments into the field, and thus only a few of these preparations are in various stages of clinical trials while none has been introduced into the market before the priority date of the present invention. From the practical standpoint these problems lead to considerable delay in bringing the potential of the beneficial microorganisms to the users. On the other hand, the solution of the present invention is based on the use of microbial extracts, lysates, or purified microbial fractions completely devoid of any viable microorganisms, which means that such mixtures of natural compounds can be used as components of the cosmetic preparations for the strengthening of the skin barrier without much regulatory limitations, provided they are not toxic (the cosmetic legislation uses the presentation of these components by the Latin name of the source microorganism followed by the attribute "ferment", e.g. *S. epidermidis* ferment).

Active substances for the first stage of the multistage preparation comprise mostly mixtures of hydrolytic enzymes with proteolytic and glycolytic activities, this composition reflects the composition of the microbial biofilms composed of individual microorganisms connected together through the adhesion proteins and covered by polysaccharide films reminding of celophane with the primary components defined as β-1,3-glucan (laminarin), β-1,4-glucan (cellulose) and a long polymer formed by β-1,4-linked N-acetyl-D-glucosamin sugar units (chitin). According to the published data, skin commensal microorganisms have the ability to dissolve pathogenic biofilms formed by the skin pathogenic bacteria through the production of proteinases, and to lesser degree laminarinases secreted by these microorganisms. The substances for the first stage composition of the preparation can be extracted from the skin commensal microorganisms, namely from the bacteria belonging to the *Staphylococcus, Streptococcus, Corynebacterium, Propionibacterium* and *Proteobacterium* species. For the purpose of the present invention the key enzymatic activities were obtained preferably from *S. epidermidis.*

In order to increase the efficiency of the product the required enzymatic activities were supplemented with those underrepresented in the commensal skin microflora, namely cellulases and chitinases. The suitable source of these enzymes is in the environmental microorganisms such as those belonging to the *Trichoderma, Pythium, Nitrosomonas* and *Mycobacterium* species, the extracts of which are commercially available. For the present invention the required enzymatic activities were obtained preferably from *Pythium nunn* (*P. nunn*) known together with *Trichoderma harzianum* as a rich source of the required enzymes (3). Technologies to obtain reproducible batches of the enzyme mixtures from the above mentioned species were solved by the inventors, as detailed in Example 1. The dosing of the enzymatic activities in the first stage composition of the preparation was first based on the literature data and then followed by the inventors' experimental data on dissolution of biofilm shown in Example 4. Consequently, the main active compounds for the first stage composition are cell-free extract, lysates and enzyme mixtures obtained from the skin commensal bacteria of *Staphylococcus, Corynebacterium, Propionibacterium* and *Proteobacterium* species and from the environmental microorganisms of *Trichoderma, Pythium, Nitrosomonas* and *Mycobacterium* species, preferably from the species *Staphylococcus* and *Pythium,* and most preferably from the microorganisms *S. epidermidis* and *P. nunn.* Active substances for the first stage composition comprise proteinases, laminarinases, celulases and chitinases.

Regarding the active substances for the second, third, and fourth stage compositions, these compounds were either identified in our experiments or they are described in studies cited in the references. Good antimicrobial activity and biofilm destruction ability was demonstrated by the laboratory tests as described in the Examples. The tests were important in determining the dilution ratios of the active substances for their incorporation into the final compositions and also brought some surprising insights, especially in their ability to reduce viability of microorganisms released from biofilms (combined effect of biofilm disruption and killing of pathogens).

Namely, the active compounds for the second stage composition were obtained from the bacterial lysates or extracts obtained from the selected bacterial species belonging to *Staphylococcus, Streptococcus, Corynebacterium, Propionibacterium* and *Proteobacterium,* preferably from *Staphylococcus* sp., most preferably *S. epidermidis.* The specific active compounds for the second stage comprise lipoteichoic acid, antimicrobial peptides of the SH-lantibiotic type, antimicrobial peptide γ-modulin, and Esp proteinase.

Active substances for the third stage composition of the preparation are cell-free extracts or lysates prepared from the environmental microorganisms selected from the families *Trichoderma, Pythium, Nitrosomonas* and *Mycobacterium,* preferably *Nitrosomonas* sp., most preferably *Nitrosomonas europea.* The extracts comprise both low molecular mass compounds and proteins, among which the most prominent position is played by the membrane complex oxidizing urea and producing nitric oxide.

The active substances for the fourth stage composition of the preparation are compounds known to contribute to the hydratation and regeneration of skin and stabilization of the normal skin microflora that can be any one or more compounds selected from a group containing xylitol, farnesol, L-arginine, safflower oil, evening primrose oil, hemp oil, rapeseed oil, wheat germ oil, lactate, glycine, fructose, niacinamide, inositol, magnesium aspartate, zinc gluconate, and copper gluconate. Any one of the listed compounds may also be included into any of the formulations for the first to third stage of the preparation.

The above described active substances and compounds can be formulated into the cosmetic or medicinal compositions and preparations. Preferably, cosmetic preparations may be formulated as skin lotions, creams, gels, or foams, while medicinal preparations may be in the form of skin emulsions, creams, foams, or gels. The form of skin lotions or skin emulsions may appear particularly suitable since they might be also easily applied as a spray.

The final preparation in the form of an application mixtures for each of the individual stage compositions contains the optimized and effective amounts of the active substances that can be easily set by an expert in the field on the basis of tests described in the present specification, or tests and data that are well known to the expert in the field. The typical effective ranges of concentrations of the active substances are provided in the Examples.

Generally, the standard application dose in case of skin emulsions or lotions is defined as 1 to 5 ml, usually as 3 ml. This dose can be easily applied using the application pump, syringe, or hand operated sprayer that may be a component of the commercial packings.

The final product comprising the multistage preparation might be sold in various commercial packaging wherein there is a choice for each individual step between, as example, 30 ml, 75 ml, 150 ml, 300 ml, 400 ml and 500 ml volumes of the product. Small volumes packages (30-150 ml) may be easily formulated into tubes, while large volume multiuser packages (300-500 ml) might be sold in bottles equipped with appropriate dispensers. Another preferred packaging can be an aerosol can, i.e. a spray.

An introduction of the chemically variable active substances into the formulation of the cosmetic or medical preparation (e.g. skin emulsion, skin milk) has appeared rather difficult. Eventually, long term trials resulted in the formulations that proved compatible with the various activities of the active substances and their mixtures, and provided the needed long term stability necessary for the commercial cosmetic and medicinal products. Finally, it proved optimal to use compositions composed of water as the solvent, triethanolamine acetate as the buffering components maintaining the beneficial acidic pH at the application sites between 4.7 and 4.8, glyceryl stearate as the emulsifier, polyacrylate crosspolymer 6 and xanthan gum as the regulators of viscosity. The stability of the composition exceeded 12 months, thus providing an indication for the expiration limits of the individual produced batches. During the introduction of the active substance mixtures into the basic oil emulsion for cosmetic formulation, a surprising enhancement of the measured values of some of the followed enzymatic activities could be observed wherein the enzymatic activities as well as the storage stabilities were up to twofold compared to the control conditions.

In the initial assessment the effects of the cosmetic compositions were verified using several groups of potential users. Considering that the primary purpose for the claimed compositions is their use in the cosmetic compositions, the obvious and fast healing effects were not anticipated, nor observed in all tested cases. Nevertheless, in all tested cases there has been a clear positive feedback from the users, in particular when compared to the numerous alternative cosmetic products for the problematic skin on the market. The complete or partial improvement of the visual appearance of the affected skin sites, cessation of subjectively unpleasant symptoms such as itching or scratching, as well as the resolution of infections by the pathogenic bacteria and fungi (staphylococci, candida) have been in particular appreciated. Further, the findings regarding the positive effects of the compositions also in individuals with skin burns, mechanical damage of the skin, where the combined effects of both cleaning and stimulation of growth may apply, are completely unexpected and original.

The preparation according to the present invention comprising four compositions (stages) is preferably applied in a sequence of four periods, wherein the first stage composition is applied in the first period, the second stage composition is applied in the second period, the third stage composition is applied in the third period, and the fourth stage composition is applied in the fourth period, wherein each of the first, second, and third period typically last for one to two weeks, and the fourth period typically lasts from one to six weeks. The application is typically performed twice a day, in the morning and in the evening, by spraying or spreading of the corresponding formulation onto the affected locations and their surroundings, or in case of very sensitive affections only onto the surrounding of the affected areas.

### BRIEF DESCRIPTION OF THE DRAWINGS

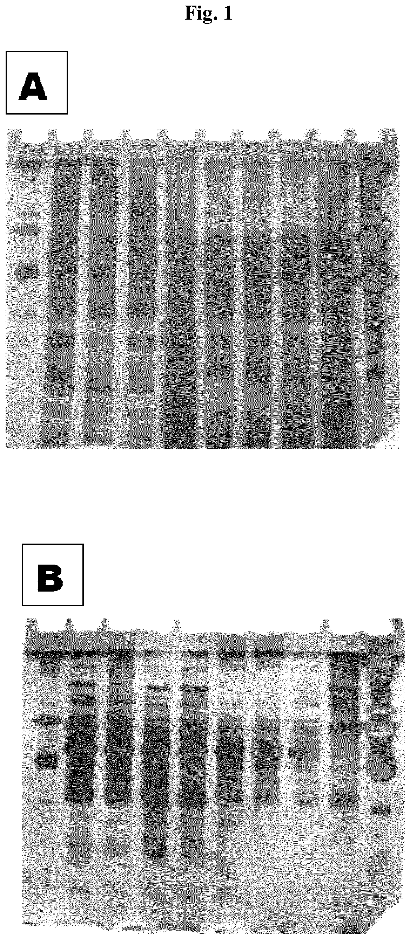

The invention is further explained using the Examples and the enclosed drawings, wherein
**Fig. 1** shows the description of the preparation of the active substances for cleaning and disruption in the first stage, specifically
   **Fig. 1A** shows the analyses of the individual fractions of proteins secreted by bacterium S. *epidermidis* strain ATCC_12228 after their concentration and transfer into the conservation buffer for the introduction into the formulation, wherein lanes 1 and 10 show the molecular standards and lanes 2 to 9 show eight different independent batches;
   **Fig. 1B** shows the analysis of proteins secreted by the environment microorganism *P. nunn* strain CBS_808.96 after their concentration and transfer into the conservation buffer for the introduction into the formulation, wherein lanes 1 and 10 show the molecular standards and lanes 2 to 9 show eight different protein batches;

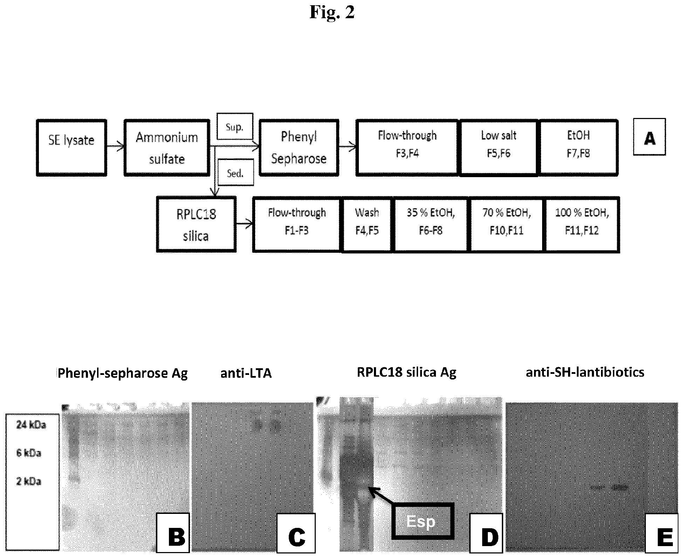

**Fig. 2** shows the method of preparation and concentration of active substance used for the calming of the immune system in the second stage, specifically
   **Fig. 2A** shows the scheme for the fractionation of bacterial lysates from *S. epidermidis* strain ATCC_12228 using ammonium sulfate precipitation, after which the soluble compounds in the supernatant ("sup") were further separated on phenyl-Sepharose® column, while the substances in the sediment ("sed") were further separated on octadecyl silica column. The fractions separated according to these schemes are abbreviated as "F".
   **Fig. 2B** to **2E** show the electrophoretic and immunochemical analyses of the separated compound analyzed by 20 % polyacrylamide electrophoresis in the presence of sodium dodecyl sulfate, wherein **Fig. 2B** and **Fig. 2C** show the compounds recovered from the supernatant after ammonium sulfate precipitation, where **Fig. 2C** specifically relates to the lipoteichoic acid (LTA), while **Fig. 2D** and **Fig.2E** show the compounds recovered in the sediment after ammonium sulfate precipitation, where **Fig. 2E** specifically relates to the lantibiotic type antimicrobial peptides. The approximate molecular mass of the analyzed compounds is indicated at the right side of the gel. The fractions F1 and F2 not retained on the octadecylsilica column were also analyzed in 12 % polyacrylamide / sodium dodecyl sulfate gels, wherein in **Fig. 2D** the band corresponding to **Esp** proteinase is marked by an arrow.

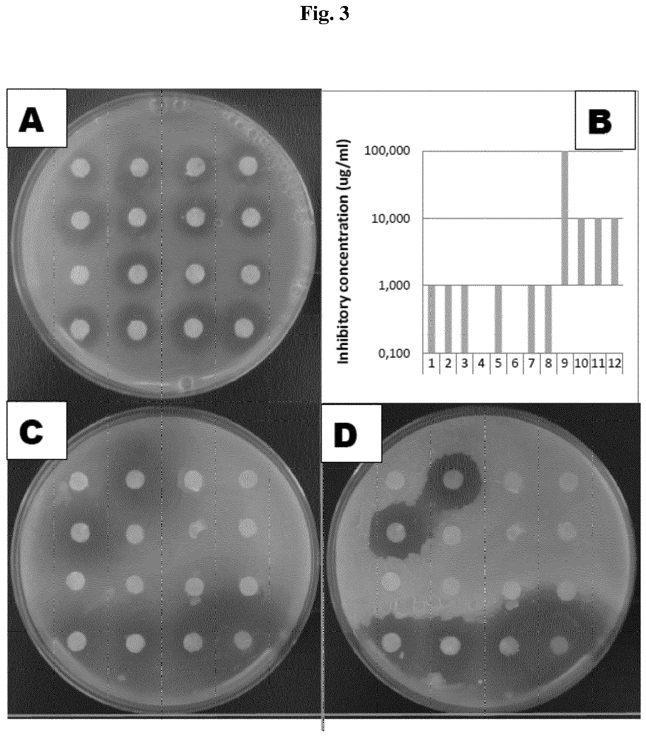

**Fig. 3** shows the laboratory tests of the antimicrobial efficiency of the prepared active substances towards the critical pathogens causing problems at the skin and mucosal surfaces, specifically
   **Fig. 3A** shows the disc test for antimicrobial efficiency towards the pathogenic bacteria S. *aureus* strain ATCC_6538 wherein the substances applied onto the individual discs were as follows: in the first row from left to right the tested substances for the first stage SEF2K1 sequentially diluted 10x, 10²x, 10³x and 10⁴x, in the second row the tested substances for the second stage SELY diluted 10²x, 10³x, 10⁴x and 10⁵x, in the third row the tested substances for the third stage NIE1 diluted 10x, 10²x, 10³x and 10⁴x, the last row contained the positive control antibiotic tetracycline with a concentration of 0,5 µg/ml, 1 µg/ml, 2 µg/ml and 4 µg/ml. 5 µl of the tested substances were applied to all discs.
   **Fig. 3B** shows the quantitative minimum inhibitory concentration (MIC) test for four different batches of the substances for the first stage SEF2 (samples 1 to 4), four different batches of the substances for the second stage SELY (samples 5 to 8), and four different batches NIE1, NIE2, NIE3 and NIE4 of extracts from the industrial wastewater cleaning bacterial communities containing *Nitrosomonas* species (samples 9 to 12) used for the third stage .
   **Fig. 3C** and **Fig. 3D** shows the disc efficiency tests of the active compounds towards the pathogenic yeast *Candida albicans* strain ATCC_10231, wherein the Petri dish shown in **Fig. 3C** was incubated for 24 h at 30 °C and the same Petri dish shown in **Fig. 3D** was incubated for 48 h at 30 °C. The volumes, compounds, and concentrations applied were identical to **Fig. 3A** except in the *last row* in which the chemical antifungal enilconazole was applied in concentrations 1 mg/ml, 2 mg/ml, 4 mg/ml and 8 mg/ml.

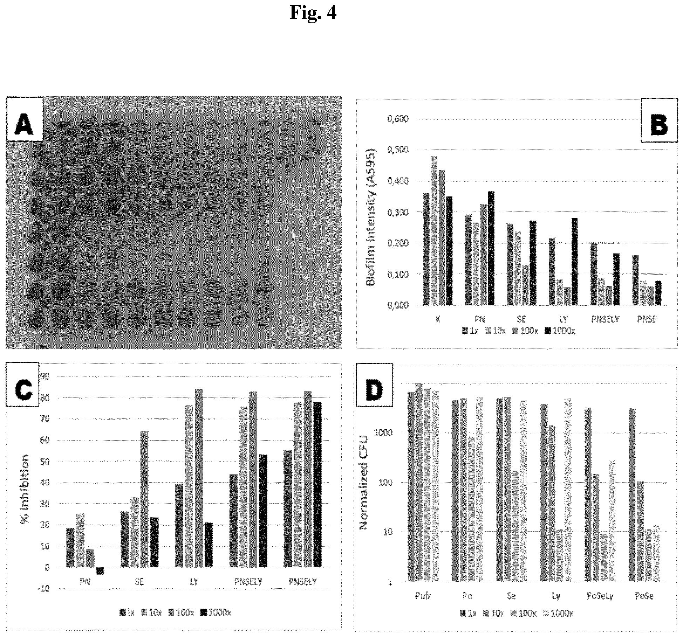

**Fig. 4** shows the results of the laboratory test of inhibition / disruption of biofilm formed by S. *aureus* ATCC_6538, specifically
   **Fig. 4A** shows an example of the microtiter plate after incubation of the formed biofilm with the tested compounds for 24 h at 30 °C, washing, and the detection of the remaining biofilm using crystal violet. Individual compound were tested in quadruplicates applied in 2 x 2 well squares in the neighbouring wells.
   **Fig. 4B** shows the averaged values of the quadruplicate assessments as shown in **Fig. 4A****,** wherein the biofilm intensity was measured in the conservation buffer only (C), or in the conservation buffer containing the compounds PNF1K1 secreted by *P. nunn* (PN), or in the conservation buffer containing the compounds SEF2K1 secreted by *S. epidermidis* (SE), or in the conservation buffer containing the *S. epidermidis* lysate SELY (LY), or in the conservation buffer containing the indicated combinations of the tested compounds (PNSELY, PNSE) using the tenfold serial dilutions (1x, 10x, 100x, 1000x).
   **Fig. 4C** presents data identical to those in **Fig. 4B** but expressed as the % inhibition of the biofilm formation based on the formula % inhibition = [(A₅₉₅ experiment - A₅₉₅ background) / (A₅₉₅ control - A₅₉₅ background)] x 100,
   **Fig. 4D** shows the viability of the pathogenic bacterium *S. aureus* strain ATCC_6538 determined as CFU (colony forming units) after cultivation on recommended media where supernatants containing the bacteria released during the dissolution of biofilms were examined before the washing step in the protocol shown in Fig. 4A, the determined number of the viable bacteria was normalized per 10000 of the released bacteria.

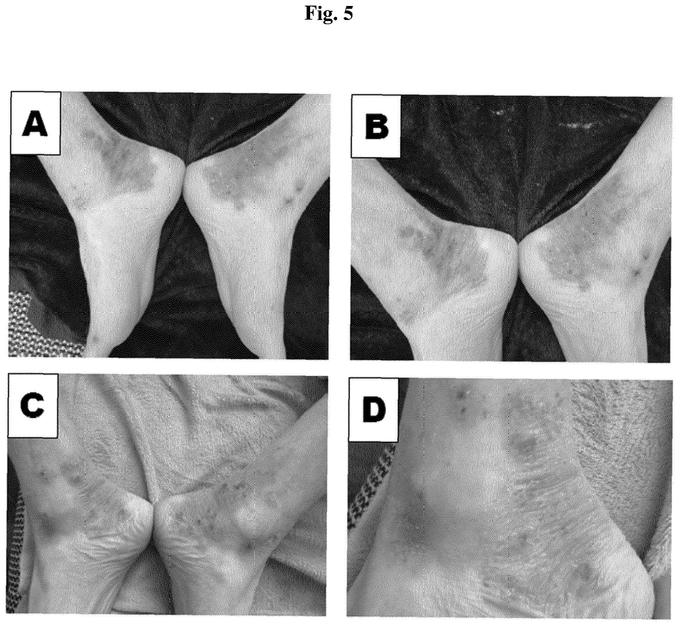

**Fig. 5** shows the results of the application of the four stage preparation according to invention in case of a proband (man, 29 years) with atopic dermatitis localized at the ankle for the entire life of proband. Shown are photographs before the beginning (panels **A** and **B**) of the application, and after 4 weeks (panels **C** and **D**) of the application of the preparation, the composition for each stage being applied for one week. The state of the affection was characterized by the proband as significant calming.

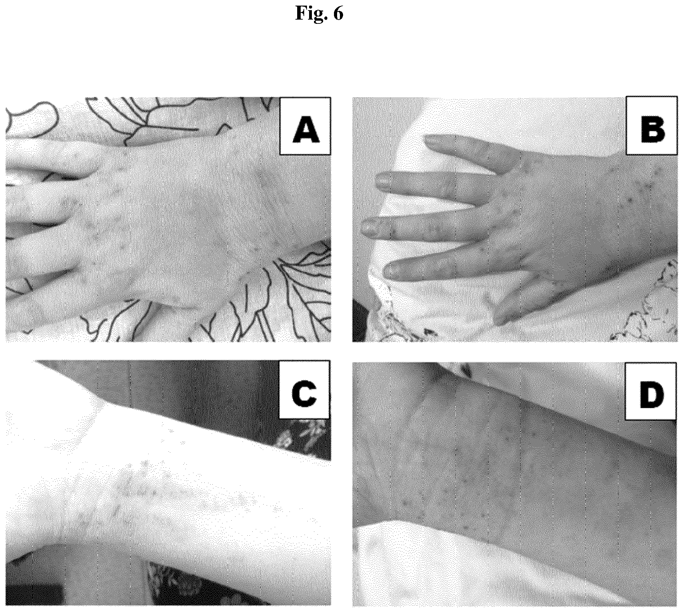

**Fig. 6** shows the result of the application in case of a proband (woman, 22 years) suffering by atopic dermatitis on her hands and forearms. Photographs show the status before the application (panels **A** and **B**) and after 4 weeks of application (panels **C** and **D**) of the four stage preparation according to the invention. The state of the affection was characterized by the proband as moderate improvement accompanied by a significant calming of the affected sites and a complete cessation of burning and itching,

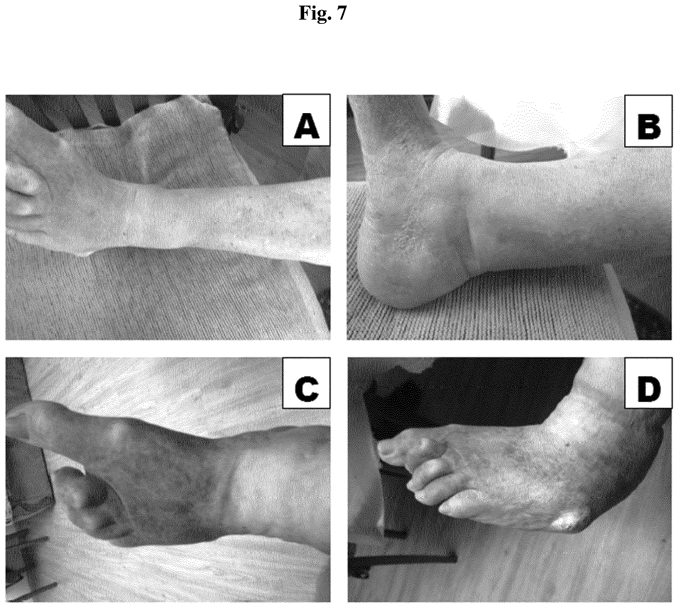

**Fig. 7** shows the course of application in case of a proband (woman, 74 years) with recurrent fungal and yeast infections on the feet and the instep. The photographs show the status before the application (panels **A** and **B**) of the product and after two weeks of applications (panels **C** and **D**) involving the consecutive application of the composition for stage one and two, respectively. The state of the affection was characterized by the proband as a significant improvement contrary to the unsuccessful application of the standard creams and ointments available on the market.

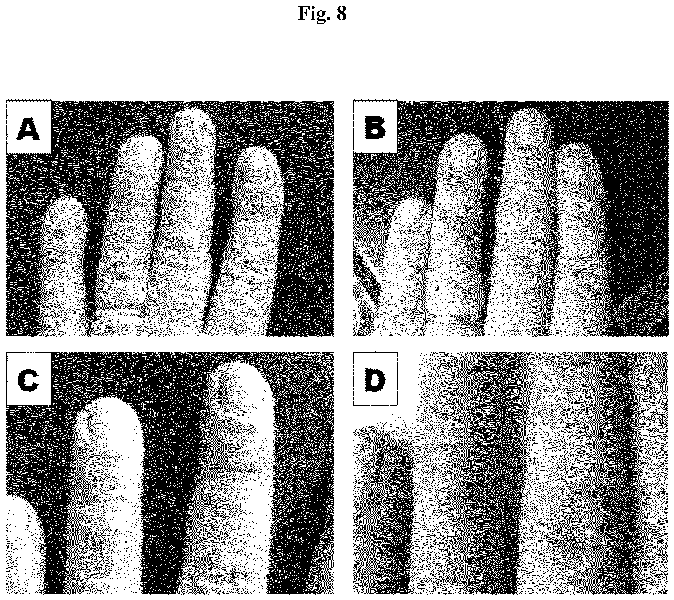

**Fig. 8** shows the course of the application of the first stage composition in case of a proband (man, 58 years) with burns on his hand. The composition was applied twice daily onto the ring finger with more severe burns using the middle finger as a control. Photographs before application (panel **A**), and 3 days, 7 days and 12 days after the beginning of the application (panels **B** to **D,** respectively) are shown. A complete healing of the skin was observed at the same time for both the fingers without any scars, coloration or other damage of the skin.

### EXAMPLES OF THE INVENTION

The invention is further demonstrated using the examples involving the biotechnological method used for the preparation of the active substances, the performed laboratory tests of the antimicrobial properties towards the critical pathogens on the skin and the mucosal surfaces, as well as practical examples of applications for some selected of skin problems potentially related to the dysbiosis of the skin microbiota. Examples are provided in order to demonstrate the principles, the substance and the practical verification of the present invention, but the invention is by no means limited only to the embodiments provided in the below given examples. The examples are provided to present the nature of the invention thoroughly and fully, and thus to justify the scope of the claims.

The examples are mostly based on methods and procedures that are common and known to the expert in the field. In order to culture and amplify the used microbial strains, the standard media were used but also some new media have been tested to suit better to the purpose of the individual cultivations. The cultures themselves were processed at various scales, from small cultures incubated in the laboratory shakers to fermentor cultures performed in 2 x 5 L laboratory scale fermenters. After the end of the culture, the cells were separated from the culture medium using a continuous flow centrifuge, and the sedimented biomass was used for the preparation of the bacterial lysates. The medium was initially filtered and then the clarified solution was concentrated by diafiltration (pressure filtration) through the cassettes with polyester sulfonate concentration membranes with 10 kDa molecular-weigh cut-off. The concentrated compounds were then transferred to conservation buffer containing preserving and stabilizing agents and having pH 4.7. The protein concentration was determined using the Bradford assay, proteinase activities using the azocasein method, and the glycohydrolase activities including the activity of laminarinase, cellulase, chitinase and amylase was determined by assaying the amount of the reducing sugars formed after the incubation with the respective substrates. One enzymatic unit (U) was defined for the purpose of the present invention as the amount of enzyme able to cleave 1 nmol of the substrate per minute under the given experimental conditions. This enzyme unit corresponds to one thousandth of the international enzyme unit. The bacterial biomass was lysed by the repeated sonication using the conservation buffer containing further detergents with saccharide hydrophilic component: methyl-6-O-(N-heptylkarbamoyl)-α-D-glucoside, N-oktanoyl-N-methylgluc-amine and 2-cyklohexylethyl-β-D-maltoside. The extracted mixtures of compounds were fractionated by ammonium sulfate precipitation after desalting to 75 % (w/w) concentrations, and the obtained fractions were further separated on phenyl-Sepharose and octadecyl silica columns, respectively. Laboratory efficacy tests included antimicrobial assays against the key skin pathogens using the disc and minimum inhibitory concentration (MIC, ref. 8) assays while the ability to dissolve biofilms was tested using the microplate assay (ref. 16).

For further practical tests, limited group of individuals with symptoms and problems associated with the damaged skin barriers and microbial skin dysbiosis was treated. These problems included atopic dermatitis (atopic eczema), skin infections by *S. aureus,* skin infections caused by fungi and pathogenic yeasts, as well as other damages of the skin of acute nature, such as skin burns. All tests were conducted on the basis of a signed informed consent. All data obtained during the study are anonymous, the results of the study in the form of questionnaires and the photo documentation are kept by the applicant in a secure repository.

### EXAMPLE 1

### Preparation of active compounds for the first stage dissolving the biofilms formed by the pathogenic skin microorganisms and suppressing the viability of the released pathogenic bacteria

In case of the commensal skin bacterium *S. epidermidis* the standard recommended medium (tryptone soya broth) was not optimal since it did not provide well defined composition and contained contaminating enzyme activities. An alternative medium containing a defined mixture of the necessary nutrients together with a cocktail of mineral and vitamins recommended for this bacterium was tested. In case of the oomycete *P. nunn* the standard minimal medium was satisfactory, provided that it was supplemented by the inducers of the required enzyme activities. While laminarin (an inducer of laminarinase, endo-β-1,3-glucosidase) and methylcellulose (an inducer of cellulase, endo-β-1,4-glucosidase) were used as described in the literature, the chitin as an inducer of chitinase cannot be used as such and it was used in the form of *N*-acetyl-D-glukosamin oligomers obtained by acid hydrolysis. As the starting biotechnological raw material, rough shrimp shells used for gardening purposes were used. Since this material also contained proteins, the mixture of *N*-acetyl-D-glukosamin oligomers and peptides after acid hydrolysis and neutralization was used advantageously as the inducer of both chitinases and proteinases as well as a source of nitrogen. At the end of the fermentation, the clarified medium was recovered by flowing the fermentation broth through a continuous flow centrifuge, while the sedimented microorganisms were obtained on a film inserted in a flow centrifuge cylinder. Overall, four fermentations were performed with the commensal skin bacterium *S. epidermidis* using the 5 L fermenters. The clarified cultivation medium was acidified to pH 4.7 using acetic acid, sterile filtered and frozen until further processing. The 2.5 L batches were thawed and concentrated about 25x using tangential flow filtration method with Minimate TFF cassette containing 10 kDa molecular weight cut-off polyester sulfonate membrane (PALL Inc.). The repeated cycles of dilution and concentration were then used to transfer the macromolecules to the conservation buffer described above. Finally, the protein mixture with a concentration of approximately 1 mg/ml was sterile filtered (Corning disposable filtration device with 0.22 µm cellulose acetate membrane) and stored at -20 °C until further use. The reproducibility of individual obtained protein fraction was confirmed by SDS polyacrylamide electrophoresis with silver staining of the fixed gels. Identical protein profile was observed for all processed batches except batch number 4 (SEF2K4), which was discarded (**Fig. 1A**). The proteins secreted by the microorganism *P. nunn* were also obtained in 8 batches having a protein concentration approximately 1 mg/ml under identical processing conditions **(****Fig. 1B**). The protein concentration and enzymatic activities of proteinases, laminarinases, cellulases, and chitinases were determined in the final mixtures. These assays confirmed very good batch-to batch reproducibility with relative deviation approximately 10 - 20 % for the enzymatic activities and about 30 % for the protein concentrations. For the first stage of application, protein concentration is monitored as well as the enzyme activites providing the following amounts in the final application formulation: 15 µg/ml of protein, 0.23 mU/ml of acidic proteinases, 0.56 mU/ml of laminarinase, 0.27 mU/ml of cellulase, and 0.47 mU/ml of chitinase.

### EXAMPLE 2

### The preparation of active compounds for the second stage calming the skin inflammation and restoring the biological protective skin barrier

In the initial trials, an oil emulsion base was supplemented with the crude bacterial extract from *S. epidermidis* strain ATCC_12228 and crude γ-modulin modified by *N*-terminal formylation after the peptide synthesis. Nevertheless, since this crude composition caused undesired reaction on the skin of users weakened by dysbiosis, further purification of the crude mixture was necessary. The adopted purification scheme is shown in **Fig. 2A** and included the precipitation of the detergent extract using 3 mol/L ammonium sulfate (final concentration, 75 % saturation) followed by a separated processing of the supernatant and the sediment by reverse phase column chromatographies (phenyl-Sepharose, octadecyl silica) as indicated. The supernatant after ammonium sulfate precipitation was filtered and directly applied onto a column of phenyl-Sepharose (1.6 x 11 cm, 22 ml) equilibrated with 3 mol/L ammonium sulfate in the presence of 0.05 mol/L sodium acetate buffer pH 4.7. After the application of the sample, the column was washed with identical buffer and then eluted with the same acetate buffer without the ammonium sulfate and thereafter with 80 % ethanol in the identical acetate buffer.

The material precipitated by ammonium sulfate was dissolved in the sodium acetate buffer and the concentration of ammonium sulfate was adjusted to 1 mol/L on the basis of conductometry. The material was spun, filtered and applied onto a column of octadecyl silica (1.6 x 13 cm, 26 ml, degree of modification by octadecyl was 20 - 22 %, particle size 60-130 µm) equilibrated in the same buffer. After sample application and washing, the column was eluted with the acetate buffer without ammonium sulfate followed by a linear gradient from acetate buffer to 95 % ethanol in acetate buffer, and fractions were collected 10 ml each. Fractions from both chromatographies were analyzed in 20 % SDS polyacrylamide gels developed by silver staining and immunoblot. For immunoblotting, the compounds separated in the polyacrylamide gels were transferred onto PVDF membrane (0,2 µm) that was blocked with defatted milk, incubated with the primary antibody labelled with biotin, washed, incubated with the streptavidin-peroxidase and developed using 3-methyl-2-benzothiazolinonhydrazonu and 4-chloro-1-naphthol resulting in the formation of brown-red unsoluble complex (**Fig. 2B** to **2E**). 12 % polyacrylamide gel was used for the detection of Esp proteinase because of the larger size of this molecule. The quantitative estimation of the compounds was performed using a comparison with the available standards (not shown) after photodocumentation and quantitative evaluation of the image. γ-modulin as a raw product of peptide synthesis comprising about 75 % of the target peptide was purified on phenyl-Sepharose column using the published method (16) and its concentration determined spectrophotometrically using the predicted extinction coefficient (ProtParam tool at web.expasy.org). The concentration of Esp proteinase was estimated directly from the intensity of the signal on the gel. The application formulation for the second stage was prepared in a similar way as described for the first stage, and the second stage formulation thus contained the following 4 active substances in approximate concentrations as follows: lipoteichoic acid from *S. epidermidis* - 10 µg/ml, specific Esp proteinase from *S. epidermidis* - 5 µg/ml, SH-lantibiotic peptides from *S. epidermidis* - 12 µg/ml, and γ-modulin from *S. epidermidis* - 38 µg/ml. Reproducibility of the preparation of the individual batches in respect to the content of the active substances was satisfactory (relative deviation up to 10 %).

### EXAMPLE 3

### Preparation of active compounds for the third stage for the restoration of the normal skin microflora

The starting material for the preparation of the bacterial extract consisted of the microbial communities used in the wastewater treatment plants, the biomass was kindly provided to the inventors by Mgr. Jakub Hejnic PhD from Centre for Applied Investigations Dobříš (CAVD) and Ing. Pavel Pícha PhD from the Institute of Water and Environment of the University of Chemistry and Technology (VSCHT) Prague. The content of the bacteria belonging to *Nitrosomonas* family deprived from the human skin of modern population due to the use of soaps and detergents (www.aobiome-com) was estimated at 25-30 %. The membrane complex comprising about 50 % cell protein and 90 % ubiquinone and cytochrome c oxidase was isolated as described previously (8) using freezing / thawing, clarification of the mixture by centrifugation at 20000 x *gₐᵥ* for 20 min. The clear supernatant was discarded and the membrane complex was washed several times, centrifuged at 3000 x g for 20 min and resuspended to a concentration of 5 mg / ml. The low molecular weight substances contained in the supernatant after the first centrifugation were purified on an octadecylsilicagel column as in Example 2. The fraction eluted from the column by 30% ethanol was carefully evaporated and the resulting material was added to the membrane fraction to a final concentration of 15 mg/ml. The extraction procedure was repeated four times to obtain extracts NIE1, NIE2, NIE3 and NIE4. The ability of the obtained extracts to decompose urea excreted by human skin into ammonia and further into nitrogen oxides, namely nitric oxide, was also confirmed at the specialized laboratory at the University of Chemistry and Technology Prague. The extracts were added to the third stage formulation after 1000x diluting at a final total concentration of 20 µg / ml. corresponding to enzyme concentration of approx. 78 mU/ml.

### EXAMPLE 4

### Selection of compounds for the nutrition of the skin and stabilization of the normal microflora for the fourth stage composition

Various compounds have been known to the experts in the field that are suitable for the nutrition of the skin, stabilization of the normal microflora on the skin, and suppression of the potential skin pathogens. In view of the considerable number of these compounds, some selection had to be made. We have found that compounds like xylitol, farnesol, L-arginin, safflower oil, evening primrose oil, hemp oil, rapeseed oil, wheat germ oil, lactate, glycin, fructose, niacinamide, inositol, magnesium aspartate, zinc gluconate, and copper gluconate might be the possible candidates. Considering our laboratory results, the price and availability of the above compounds, we decided to omit the farnesol and safflower oil, and to include the other components into the composition in the following concentrations (in % by weight), listed in the order of the *decreasing* abundance: xylitol 6 %, hemp oil 5 %, rapeseed oil 5 %, L-arginine 1 %, evening primrose oil 1 %, wheat germ oil 1 %. For the minor components such as lactate, glycine, fructose, niacinamid, inositol, magnesium asparate, zinc gluconate and copper gluconate, addition up to 0.1 % (by weight) was sufficient for the given purpose.

### EXAMPLE 5

### Examples of suitable cosmetic formulations compatible with the active substances and results of the stability tests

In order to assure the compatibility with the active compounds and the long-term stability of the products, various formulations mostly on the basis of stabilized oil emulsions were tested for the development of skin lotions or skin emulsions as the final products. Some simple oil emulsions could not achieve the needed microbial contamination levels or creaming (separation and flotation of the oil components) occurred after the storage for 3 months at the recommended storage temperature (15-25 °C). Using the gradual increase in the complexity of the oil emulsion and the inclusion of new emulsifiers and stabilizers helped to increase the stability up to 9 months, but very high viscosity of the composition prevented its efficient application onto the affected sites. In the final round of optimization, the use of modern emulsifiers rather than some classical ones allowed to decrease their concentration as well as the concentration of some stabilizers, providing a composition stable for more than 12 months (1 year) at the recommended storage temperature (15-25 °C). Such a composition, emulsion No. 4, was then used for all the test formulations, and has been used for the manufacture of the commercial products.

The preparation of the optimized test emulsion proceeded in a standard way in which all the water soluble components represented one phase and all the oil soluble component represented the second phase. Both phases were heated and then mixed vigorously using the laboratory or industrial large scale blenders. Under the constant vigorous mixing (10000 rpm), the mixture was allowed to cool.

The overall composition of emulsion No. 4 was as follows (all numbers are %, by weight): water 70,137, xylitol 5.0, hemp (*Cannabis sativa*) oil 5.0, rapeseed (*Brassica campestris*) oil 5.0, glycerine 4.0, urea 4.0, glyceryl stearate citrate 2.0, olive (*Olea europaea*) oil 1.0, wheat germ oil 1.0, evening primrose (*Oonothera biennis*) oil 1.0, phenoxyethanol 0.9, polyacrylate crosspolymer-6 0.25, lavender extract 0.2, L-arginine 0.2, ethylhexylglycerin 0.1, Lactil® 0.1, Sepitonic® 0.1, D,L-tocopherol (vitamin E) 0.01, coloring agent amaranth A12385 (E123) 0.003.

The common practice in the manufacture of cosmetic and medicinal preparations such as skin lotion required that the components critical for the fourth stage composition providing the nutrition and stabilization of the skin microflora be a part of the above composition. Thus it represents the final formulation for the fourth stage composition.

The first stage composition thus typically contained, in the base formed by the above formulation, 0.03-0.06 mg of proteins, 0.47-0.94 mU of proteinases, 1.13-2.26 mU of laminarinases, 0.53-1.06 mU of cellulases and 0.93-1.86 mU of chitinases per 3 ml application dose.

The second stage composition contained 8-12 µg of lipoteichoic acid of the *S epidermidis* type, 30-40 µg of antimicrobial SH-lantibiotic peptide, 16-20 µg of antimicrobial γ-modulin, and 13-21 µg of Esp proteinase from *S. epidermidis* per 3ml application dose.

Finally, the composition for the third stage contained 60 µg of the *Nitrosomonas* membrane complex (45 µg of low-molecular substances and 15 µg of protein complex) per 3ml application dose.

### EXAMPLE 6

### Laboratory tests for antimicrobial activities

Antimicrobial and biofilm dissolving activities were tested using the appropriate laboratory tests, namely the disc test on Petri dishes ("disc test"), the minimum inhibitory concentration test on microtiter plates ("MIC test") and the biofilm dissolution test in which the residual biofilm after an incubation with the test compounds is measured using crystal violet ("biofilm test", see in the next Example 7).

We first tested the ability of the active compounds and formulations to inhibit the growth of the most common bacterial skin pathogen *S. aureus.* The particular laboratory strain used (ATCC_6538) is known to represent an aggressive strain commonly used as a standard in studies of disinfections and antiseptic biocides. The most profound effects in the disc test were achieved using the secreted proteins from *S. epidermidis* diluted a hundred fold, a lysate from this bacteria diluted hundred fold, and the membrane aminooxidase / hydroxylamine oxidase complex from the environmental bacteria *Nitrosomonas* sp. (**Fig. 3A**). These results were corroborated in the MIC test performed with four different batches of the active compounds. Here, three out of four batches of the secreted *S. epidermidis* proteins inhibited the growth of the pathogen *S. aureus* at concentrations as low as 0.1 µg/ml (determined as protein), while the lysate from the same bacterium provided very similar results (**Fig. 3B**). On the other hand, the *Nitrosomonas* membrane complex provided somewhat lower activities with inhibitions at 10 µg/ml (**Fig. 3B**). Finally, the antimicrobial activities were also tested towards the yeast *Candida albicans* that might acquire aggressive fibrillar forms in individuals with problematic skin and often occurs in co-infections with the other pathogens. The fast-growing aggressive strain ATCC_10231 used commonly as the sterilization control and in the tests of antifungal compounds. In case of this yeast, only secreted proteins and lysates from *S. epidermidis* provided efficient inhibition (**Fig. 3C** and **3D**).

### EXAMPLE 7

### Demonstration of the ability to dissolve biofilms in laboratory tests

The primary purpose for the biofilm test was to prove the ability of the active compounds to dissolve the biofilms formed by pathogenic microorganisms *S. aureus* and to determine if a simultaneous killing of the released pathogens determined as the reduction of their viability could be observed. The standard assay in which the residual biofilm in the microtiter wells is stained by crystal violet and determined spectrophotometrically at 595 nm after the extraction of the dye into ethanol was used. Because of the inherent variability in this biological assay, all experiments were performed in quadruplicates and the presented data thus represent the averaged values measured in the four adjacent wells (**Fig. 4A**). The ability of the tested mixtures to dissolve biofilms was measured at four different concentrations, when the 10x or even 100x diluted mixtures often provided the best results (**Fig. 4B**). The experiments covering the combinations of the active compounds appeared critical for the assessment of the final formulations. Here, the combinations involving the soluble secreted proteins from *S. epidermidis* and *P. nunn* appeared most (PNSE, **Fig. 4B** and **4C**). Another important parameter is the viability of pathogenic microorganisms released from biofilms: if the released bacteria remain viable, there is a risk of re-colonization and new biofilm formation after application. In order to assess the effect of the compounds on the viability of the pathogenic bacteria released from the biofilms, aliquots of the collected medium after incubation with the active compounds were cultured on Petri dishes under optimal conditions for 24 h and the number of viable microorganisms was expressed. This experiment clearly demonstrated that the microbial extracts not only have the ability to disrupt the biofilm, but also to kill (or at least significantly reduce the viability) of the released pathogenic bacteria. The best effects were achieved using the mixture of secreted proteins from both microorganisms diluted 100x or 1000x (**Fig. 4D**).

### EXAMPLE 8

### Practical tests of the multistage preparations

Several rounds of practical tests were performed with the four-stage preparation formulated as the cosmetic product, skin milk (each stage active substances were formulated into basic emulsion No. 4 of Example 5).

The first round of testing was performed after obtaining a Report on the Cosmetic Product Safety. The preparation was administered in a four-week dosing regimen (each stage composition for one week in the morning and evening) in 10 healthy subjects with normal intact skin. With the exception of very mild pruritus in the first stage composition, no side or adverse reactions were observed after monthly administration. These results, together with those reported in the Safety Report, indicated the safety of the product when applied to the healthy skin of normal individuals.

Based on these results, in the second round the preparation was tested on individuals with skin problems related to dysbiosis (such as atopic dermatitis, acne, psoriasis and rosacea) or burn injury. The tests revealed (1) a greater sensitivity of these individuals to the former second stage composition of the preparation that was resolved by further purification, as described in Example 2, (2) beneficial effects even in severe cases of skin damage such as burns where the application of the cosmetic product around the burns proved salvatory in certain cases. The extensive hand burn associated with local sepsis was significantly improved after two weeks of application comprising the first and the second stage composition and the infection vanished.

In the third round of practical testing the limited group of probands prone to skin dysbiosis were subjected to a treatment using the full four stages preparation according to the invention after the informed consent.

29 year-old man reported problems with atopic dermatitis from early childhood localized in the later period in the ankle area. The problem could not be solved using a large amount of preparations available on the cosmetics market. The four stage preparation was applied for 4 weeks twice daily. In 4 weeks the proband reported a significant improvement of the problem that is confirmed by photodocumentation (**Fig. 5**). Subjectively the proband reported a significant calming effect although he found the time for absorption of the product (caused by the presence of the hydrated components) rather long.

22 year-old woman suffered by atopic eczema on her hands that could not be managed by several means available for this diagnosis. The four stage preparation was intended as above, although only stage one and stage two formulations, one week each, were applied In two weeks, the proband describes a partial resolution of the problem accompanied with a significant calming, the calming and decrease in the extent of affection is obvious from the photographical documentation (**Fig. 6**).

74 year-old woman with diabetes and kidney damage, dependent on regular dialysis three times a week, suffered by long-term problems with fungi and yeasts on her legs accompanied by the cornering and cracking of the skin in the instep and on the sole. The four-stage preparation according to the invention was applied to the proband for four weeks. After 1 month of application, a significant decrease in the extent of infection was observed together with a complete cessation of the infection on about 80 % of the affected area. The provided photodocumentation (**Fig. 7**) was in support of the subjective evaluation by the proband and the doctor.

58 year-old man reported the burns on his hand caused by a laboratory accident involving the formation of a deep, swollen burn on the ring finger of the left hand while the middle finger had a smaller injury (**Fig. 8A**). The application of the first stage composition was performed daily only on the ring finger protecting the middle finger from the application during the spraying, and the course of the healing was followed by photodocumentation (**Fig. 8B** to **Fig. 8D**). It could be observed and documented that after 12 days a nearly complete healing at both sides occurred, and only a mild redness could be observed at the sites of the original injuries (**Fig. 8D**). During the following two weeks a complete healing was finished without any scars or changes in the pigmentation on the burn fingers.

The inventors will perform practical tests under the supervision of the dermatologists with the aim to acquire a sufficient number of observations allowing the statistical evaluation as well as to get the relevant experience for the organization of more extensive clinical trials with medicinal use of the preparations.

### INDUSTRIAL APPLICABILITY

The combined multistage microbial preparation according to the present invention can be used for the manufacturing of cosmetic products, functional cosmetics or medicinal cosmetics suitable for individuals prone to skin dysbiosis such as individuals with problems with atopic dermatitis, acne, rosacea, psoriasis, vitiligo and other skin problems as well as for the relief in case of acute skin problems such as burns, scratches etc. Also, the formulations and compositions described here might be useful for skin conditioning and prevention of the skin diseases strengthening the biological component of the skin barrier. An advanced manufacturing method may also be developed producing the formulations in the sterile form for their use in the treatment of atopic dermatitis, acne, rosacea, psoriasis, vitiligo, skin damages and acute skin infections in burned patients or patients the skin of whom has been otherwise damaged.

### REFERENCES

1. Cohen AL, Yamasaki K, Sanchez KM a spol. (2010a) J Invest Dermatol 130: 192-196.
2. Cohen AL, Yamasaki K, Muto J a spol. (2010b) PLoS One 5: e8557.
3. Elad Y, Lifshitz R, Baker R (1985) Physiol Plant Pathol 27, 131-148.
4. Gallo RL, Nakatsuji T US2015/290209 A1 (published on 15th October 2015).
5. Ganju P, Nagpal S, Mohammed MH a spol. (2016) Sci Rep 6: 18761.
6. Garcia-Gomez E, Miranda-Ozuna JFT, Diaz-Cedillo F a spol. (2017) J Med Microbiol 66: 864-873.
7. Grice EA, Segre JA (2011) Nat Rev Microbiol 9: 244-253.
8. Hooper AB, Erickson RH, Terry KR (1972) J Bacteriol 110: 430-438.
9. Human Microbiome Project Consortium (2012) Nature 486, 207-214.
10. Iwase T, Uehara Y, Shinji H a spol. (2010) Nature 465: 346- 349.
11. Kazue T, Makioka Y CN2018/107922956 A (published on 17th April 2018).
12. Kleinberg I, Zhang Z US2016/263154 A1 (published on 15th September 2016).
13. Kobayashi T, Glatz M, Horiuchi K a spol. (2015) Immunity 42, 756-766.
14. Kong HH, Oh J, Deming C a spol. (2012) Genome Res 22: 850-859.
15. Lai Y, DiNardo A, Nakatsuji T a spol. (2009) Nat Med 15, 1377-1386.
16. McKevitt AI, Bjornson GL, Mauracher CA a spol. (1990) Infect Immun 58, 1473-1475.
17. Naik S, Bouladoux N, Wilhelm C a spol. (2012) Science 337: 1115-1119.
18. Nakatsuji T, Chen TH, Narala S a spol. (2017) Sci Transl Med 9: eaah4680.
19. Nakatsuji T, Gallo RL US2018/289751 A1 (published on 11th October 2018).
20. Park TH, Kim SH, Jin YJ, An SS, Lee JH KR2017/3478 A (published on 9th January 2017)
21. Sanford JA, Gallo RL (2013) Semin Immunol 25, 370-377.
22. Smoragiewics W, Karska-Wysocki B, Bazo M US2011/0195057 A1 (published on 11th August 2011).
23. Sugimoto S, Iwamoto T, Takada K a spol. (2013) J Bacteriol 195: 1645-1655.
24. Takayama K, Makioka Y CN2018/107922956 A (published on 17th April 2018).

## Claims

1. A combined multistage microbial preparation **characterized in that** it is composed of four different compositions for sequential application onto skin, where
the first stage composition comprises active substances able to dissolve biofilms formed by skin pathogens and suppress the viability of the pathogenic microorganisms released from the biofilms, the active substances being cell-free extracts or lysates prepared from any of the commensal skin microorganisms selected from the bacteria of the genera *Staphylococcus, Streptococcus, Corynebacterium, Propionibacterium* and *Proteobacterium,* and any of the environmental microorganisms selected from the genera *Trichoderma, Pythium, Nitrosomonas* and *Mycobacterium;*
the second stage composition comprises active substances able to calm the inflammation and restore the biological skin barrier, the active substances being cell-free exctracts or lysates prepared from any of the commensal skin microorganisms selected from the bacteria of the genera *Staphylococcus, Streptococcus, Corynebacterium, Propionibacterium* and *Proteobacterium;*
the third stage composition comprises active substances helping to restore the normal skin microflora, the active substances being cell-free extarcts or lysates prepared from any environmental bacteria selected from the genera *Nitrosomonas* and *Mycobacterium;*
and the fourth stage composition comprises active substances providing nutrition to the skin and further supporting the colonization by commensal skin microorganisms while simultaneously suppressing the pathogens, in which the active substance is at least one compound selected from the group consisting of xylitol, farnesol, L-arginine, safflower oil, evening primrose oil, hemp oil, rapeseed oil, wheat germ oil, lactate, glycine, fructose, niacinamide, inositol, magnesium aspartate, zinc gluconate, and copper gluconate.

2. The combined multistage microbial preparation according to Claim 1 **characterized in that** the cell-free extracts or lysates of commensal skin bacteria are prepared from *Staphylococcus epidermidis* and the extarcts or lysates prepared from the environmental microorganisms are prepared from the oomycete *Pythium nunn* and/or bacterium *Nitrosomonas europea.*

3. The combined multistage microbial preparation according to claim 1 or 2 **characterized in that** the first stage composition contains 0.47-0.94 mU of proteinases, 1.13-2.26 mU of laminarinases, 0.53-1.06 mU of cellulases and 0.93-1.86 mU chitinases per 3 ml application dose.

4. The combined multistage microbial preparation according to claim 1 or 2 **characterized in that** the second stage composition contains 8-12 µg of lipoteichoic acid, 30-40 µg of antimicrobial SH-lantibiotic peptides, 16-20 µg of the antimicrobial γ-modulin and 13-21 µg of Esp proteinase per 3 ml application dose.

5. The combined multistage microbial preparation according to claim 1 or 2 **characterized in that** the third stage composition contains 60 µg of the substance mixture from *Nitrosomonas europea* per 3ml application dose, the substance mixture consisting of about 45 µg of low-molecular substances and about 15 µg of protein complex comprising urea oxidizing and nitric oxide producing membrane complex.

6. The combined multistage microbial preparation according to any of claims 1 to 5 for use as the cosmetic preparation.

7. The combined multistage microbial preparation according to claim 6 for use as the cosmetic preparation for calming and regenerating irritated or burned skin and/or strengthtening the biological component of the skin barrier and maintaining normal skin microflora in good condition.

8. The combined multistage microbial preparation according to any claim 1 to 5 for use as the medicinal preparation.

9. The combined multistage microbial preparation according to claim 8 for use as the medicinal preparation for the treatment of skin diseases or injuries.

10. The combined multistage microbial preparation according to claim 9 for use in the treatment of atopic dermatitis, acne, rosacea, psoriasis, vitiligo or skin damage due to the mechanical injury or burning.

11. The combined multistage microbial preparation according to any claims 1 to 10 for sequential application in four different periods wherein in the first period the first stage combination is applied, in the second period the second stage combination is applied, in the third period the third stage combination is applied and in the fouth period the fourth stage combination is applied, wherein each of the first, second, and third period lasts for one to two weeks and the fourth period lasts for one to six weeks.

12. The combined multistage microbial preparation according to any of the previous claims 1 to 11 **characterized in that** the composition for any of the stages is formulated into any of the application forms selected from skin emulsion, cream, ointment, gel, foam, skin lotion or any cosmetic or pharmaceutical composition containing the oil component.

13. The combined multistage microbial preparation according to claim 12 **characterized in that** the composition of each stage contains water as the solvent, olive oil, as the oil components, citric acid or acetic acid or lactic acid or succinic acid buffered with triethanolamine as the buffering components; glycerine and urea as the skin conditioning agents; phenoxyethanol, ethylhexylglycerin and tocopherol as the conservants; amaranth as the coloring agent; glyceryl stearate and cetylalcohol as the emulsifiers; polyacrylate crosspolymer-6 and xanthan gum as the viscosity regulators; and lavender oil as the perfume component.

14. The method of cosmetic treatment **characterized in that** the combined multistage prepation according to any of claims 1 to 13 is applied sequentially in four periods, wherein in the first period the first stage composition is applied, in the second period the second stage composition is applied, in the third period the third stage composition is applied and in the fouth period the fourth stage composition is applied wherein each of the first, second, and third period lasts for one to two weeks and the fourth period lasts for one to six weeks.

15. The method of treatment according to claim 14 **characterized in that** application is done at least once a day, preferably twice a day, in the morning and in the evening, by spraying or spreading of the application dose of the corresponding stage composition on the affected sites or around the affected sites, or in case of very sensitive foci only in the surrounding of the affected sites.
